# EUROPEAN PATENT APPLICATION

(11) **EP 1 852 106 A1**
(43) Date of publication of application: **07.11.2007**
(21) Application number: 06713464.3
(22) Date of filing: 10.02.2006
(51) Int. Cl.: A61K 9/06, A61K 47/14, A61K 47/44, A61P 27/02, A61M 35/00

(54) **SOLID OPHTHALMIC DRUG FOR EXTERNAL USE**

(30) Priority: 17.02.2005 JP 2005041368
(71) Applicant: Senju Pharmaceutical Co., Ltd., Osaka-shi, Osaka 541-0046 (JP); Tojo, Kakuji, Iizuka-shi, Fukuoka 820-0066 (JP)
(72) Inventor: KIMURA, Chiharu, ushu-shi Fukuoka, 8070851 (JP)
(74) Representative: von Kreisler Selting Werner
(86) International application number: PCT/JP2006/302321
(87) International publication number: WO 2006/087968

(57) **Abstract**

An ophthalmic solid pharmaceutical preparation for external use is disclosed which can be used to continuously administer with ease a pharmacologically active agent to ocular local tissues. The pharmaceutical preparation is a solid pharmaceutical preparation containing a pharmacologically active ingredient in a base, which may comprise an oily base, and designed to be applied by being rubbed on the surface of the skin including the surface of either of the eyelids to deliver, through the skin of the eyelids, the pharmacologically active ingredient to the local tissues of the eye located on the backside of the eyelids.

## Description

### TECHNICAL FIELD

The present invention relates to a novel form of ophthalmic pharmaceutical preparation for external use, more specifically to an ophthalmic solid pharmaceutical preparation for external use, and in particular to an ophthalmic solid pharmaceutical preparation for external use, which solid pharmaceutical preparation is so designed that a portion of it is applied to the surface of the skin including the front surface of the eyelids by rubbing it directly on the surface of the skin to let its pharmacologically active ingredient be transferred, through the very area of the skin, to ocular local tissues such as the conjunctiva, cornea and the like.

### BACKGROUND ART

Among ophthalmic pharmaceutical preparation forms for external use, i.e., for topical application to the eye, the most popular has been eye drops, with ophthalmic ointment following them. Both of these forms of pharmaceutical preparations are designed to bring the pharmaceutical preparations into direct contact with the cornea or conjunctiva of the eye to let their active ingredients be transferred to such regions. Though eye drops are easy to apply and excellent in its quickness of action, they are readily washed away by the tear fluid. Therefore, when the local concentration of a pharmacologically active ingredient is to be maintained for an extended length of time, their frequent instillation to the eye is sometimes required. While most of the eye drops contain one or more preservatives in order to prevent growth of microorganisms, preservatives are likely to cause irritation to the conjunctiva and cornea. Further, eye drops containing a preservative are subject to a limitation that they cannot be applied immediately before going to sleep, for the turnover of the tear fluid being lost when one is sleeping, eye drops applied are thus not washed away by the tear fluid. On the other hand, while ophthalmic ointment is as excellent as eye drops in its quickness of action, with, in general, somewhat longer duration of effect compared with eye drops, it has such drawbacks as the difficulty encountered by a patient when he tries to apply it by himself, blurred vision it causes for some length of time after application by covering the tear film, and sticky eyelid edges it causes, and the like.

One of new approaches proposed with regard to ophthalmic pharmaceutical preparation forms for external use is a transdermal treatment system consisting of a support layer and a pressure-sensitive adhesive reservoir layer made of a polymeric material containing pilocarpine (Patent Document 1). This is a system by which it is intended to let pilocarpine be transferred to the eye by way of the blood stream which has received pilocarpine absorbed through the skin on the surface of which the system is affixed.

Further, in order to deliver a drug to the posterior ocular tissues, such as the lens, vitreous body, choroid, and retina, to which a pharmacologically active ingredient in general is hardly accessible either by topical instillation or subconjunctival injection, as well as for easier guarantee of patients' compliance, there has also been proposed an ophthalmic transdermal patch comprising a drug containing layer which homogeneously contains a drug and a transdermal absorption enhancer in a base matrix (Patent Document 2).

One method for the treatment of dry eye syndrome has also been proposed based on a transdermal patch or pad (Patent Document 3).

Further, a transdermal absorption-type preparation for the treatment of ophthalmic diseases has been proposed, which, having on a support layer a plaster layer containing a drug, is applied to the surface of the skin including the front surface of the eyelids (Patent Document 4), and can transdermally deliver a drug for treating ophthalmic diseases to the outer segments of the eye including the conjunctiva, lacrimal tissues, and cornea in relatively short time and allow it to continuously exhibit its pharmacological effects. This preparation allows one to administer a drug to the local tissues of the eye through the skin and substantially not via the circulation of the blood, by affixing it to the surface of the skin including the front surface of the eyelids, which are adjacent to the outer segments of the eye.

However, any of the above forms of preparations, which are used by being affixed to the skin, includes, as an essential component, a sheet of cloth or some other supporting member which supports the layer containing a drug, and, therefore, the preparation must be kept on the skin together with the support member all through the time during which the administration of the drug is intended. In order to let the drug be transferred to the ocular tissues of interest as selectively as possible, it is necessary to affix the preparation to the surface of the skin including the surface of the eyelids. However this is not only eye-catching and looks clumsy, but also causes problems to the user such as a sense of discomfort, or hindered motions of the eyelids (opening and closing of the eyelids, blinking), depending on the position at which it is applied, as well as its size.
[Patent Document 1] Japanese Patent Application Publication H8-509716
[Patent Document 2] WO 01/26648
[Patent Document 3] USP 6277855
[Patent Document 4] WO 2004/064817

### DISCLOSURE OF INVENTION

### THE PROBLEM TO BE SOLVED BY THE INVENTION

Against the above background, the purpose of the present invention is to provide a novel type of method for supplying a pharmacologically active ingredient to ocular local tissues, and also a novel form of an ophthalmic pharmaceutical preparation for external use, which is free of the above problem in appearance during application, causes no sense of discomfort on the eyelids in the user, or hinders no motion of the eyelids, and enablers continuous administration of a pharmacological ingredient with great ease.

### [The means to solve the Problem]

The present inventors, as a result of investigations for the above purpose, found that a solid pharmaceutical preparation which is prepared so as to contain a pharmacologically active ingredient, using a material which is capable of forming a solid that is soft enough for application by rubbing it on the skin, could be held in hand and applied by rubbing on the surface of the skin including the front surface of either of the eyelids, thereby enabling continuous administration of it to the anterior segment of the eye through the skin of the eyelids. Thus, the present invention provides what follows.
(1) An ophthalmic solid pharmaceutical preparation for external use comprising a base containing a pharmacologically active ingredient, wherein the solid pharmaceutical preparation is designed to be applied by rubbing on the surface of the skin including the surface of either of the eyelids to deliver, through the skin of the eyelids, the pharmacologically active ingredient to ocular local tissues located on the backside of the eyelids.
(2) The ophthalmic solid pharmaceutical preparation for external use according to (1) above, wherein the base comprises an oily solid base.
(3) The ophthalmic solid pharmaceutical preparation for external use according to (2) above, wherein the oily solid base is wax or vaseline.
(4) The ophthalmic solid pharmaceutical preparation for external use according to (3) above, wherein the wax is animal wax or vegetable wax.
(5) The ophthalmic solid pharmaceutical preparation for external use according to one of (1) to (4) above, wherein the preparation contains a skin permeation enhancer.
(6) The ophthalmic solid pharmaceutical preparation for external use according to (5) above, wherein the skin permeation enhancer is selected from the group consisting of an ester made from a fatty acid and a lower aliphatic alcohol, and a surfactant.
(7) The ophthalmic solid pharmaceutical preparation for external use according to (6) above, wherein the content of the ester is 30-60 wt%.
(8) The ophthalmic solid pharmaceutical preparation for external use according to (6) or (7) above, wherein the ester is isopropyl myristate or isopropyl palmitate, and wherein the surfactant is a nonionic surfactant.
(9) The ophthalmic solid pharmaceutical preparation for external use according to one of (1) to (8) above, wherein the base comprises an oily solid base, wherein the content of the oily solid base in the pharmaceutical preparation is 15-60 wt%.
(10) The ophthalmic solid pharmaceutical preparation for external use according to one of (1) to (9) above, wherein the pharmacologically active ingredient is selected from the group consisting of non-steroidal antiinflammatory agents, antiallergic agents, anti-glaucoma agents, anti-cataract agents, antimicrobial agents, antiviral agents, antifungal agents, antibiotics, sulfa agents, steroidal antiinflammatory agents, miotic agents, mydriatic agents, local astringents, vasoconstrictors, anticholinesterases, surface anesthetics, and vitamins.
(11) The ophthalmic solid pharmaceutical preparation for external use according to one of (1) to (10) above, wherein the shape of the pharmaceutical preparation is of a short bar.
(12) A stick type ophthalmic preparation comprising a generally cylindrical container body defining one openable end, a movable bottom member which is fit in the interior of the container body and can be translated in the direction toward the openable end, and a drive means to cause the movable bottom member to translate in the direction toward the open end, wherein the ophthalmic solid pharmaceutical preparation for external use according to one of (1) to (11) above is contained, in the container body, between the openable end and the movable bottom member, wherein the ophthalmic solid pharmaceutical preparation for external use can be projected from the container body as a result of the forward translation of the movable bottom member driven by the drive means.
(13) A method for delivering a pharmacologically active ingredient to the ocular local tissues located on the backside of the eyelids, the method comprising rubbing an ophthalmic solid pharmaceutical preparation for external use comprising a base containing a pharmacologically active ingredient, on the surface of the skin including the surface of either of the eyelids to apply the preparation.
(14) The method according to (13) above, wherein the base comprises an oily solid base.
(15) The method according to (14) above, wherein the oily solid base is wax or vaseline.
(16) The method according to one of (13) to (15) above, wherein the ophthalmic solid pharmaceutical preparation for external use contains a skin permeation enhancer.
(17) Use of an oily solid base for the manufacture of an ophthalmic solid pharmaceutical preparation for external use containing a pharmacologically active ingredient for delivering the pharmacologically active ingredient to the tissues located on the backside of the eyelids.
(18) Use of an oily solid base and a skin permeation enhancer for the manufacture of an ophthalmic solid pharmaceutical preparation for external use containing a pharmacologically active ingredient for delivering the pharmacologically active ingredient to the tissues located on the backside of the eyelids.
(19) The use according to (17) or (18) above, wherein the oily solid base is wax or vaseline.

### THE EFFECT OF THE INVENTION

Holding in hand and rubbing it on the surface of the skin including the front surface of the eyelids, the ophthalmic solid pharmaceutical preparation for external use of the present invention can be used to apply a pharmacologically active ingredient, together with its base, to the surface of the skin. The pharmacologically active ingredient contained in the pharmaceutical preparation thus applied permeates across the skin and efficiently reaches ocular local tissues located on the side where the application was made, such as the conjunctiva, cornea and the like, not through the systemic distribution by the blood circulation or via the metabolism of the pharmacologically active ingredient. Thus, the present invention achieves delivery of a pharmacologically active ingredient to ocular local tissues, and enables highly continuous delivery of it and long lasting pharmacological activity, while avoiding risks of side effects that would be encountered with systemic administration of a pharmacologically active ingredient. Further, the present invention does not require such a support member as needed in the case of a patch-type preparation, its application, though being made to the surface of the skin including the front surface of the eyelids, neither gives clumsiness in appearance or a sense of discomfort to its user, nor hinders eyelids' motion. Thus, it can be applied anywhere in daily life, thereby serving to improve the compliance of the patient and to let the effect of the pharmaceutical preparation be fully expressed. In addition, it allows to control the length of time during which its pharmacological effect lasts, by adjusting the number of times the pharmaceutical preparation is rubbed on the skin, e.g., 1-10 times.

Also, according to the present invention, contact of a high concentration of a pharmacologically active agent with the cornea and conjunctiva, as is common in the case of eye drops or ophthalmic ointment, is avoided, and it allows a pharmacologically active agent to gradually permeate through the skin of the eyelids. Therefore, even a pharmacologically active agent that is found irritative at higher concentrations could be employed.

### BRIEF DESCRIPTION OF DRAWINGS

[FIG. 1] Fig. 1 illustrates a side view of a vertical diffusion cell.
[FIG. 2] Fig. 2 illustrates a graph showing the result of an in vitro skin permeation test 1.
[FIG. 3] Fig. 3 illustrates a graph showing the result of an in vitro skin permeation test 2.
[FIG. 4] Fig. 4 illustrates a graph showing the result of an in vitro skin permeation test 3.
[FIG. 5] Fig. 5 illustrates a graph showing the result of an in vitro skin permeation test 4.
[FIG. 6] Fig. 6 illustrates a graph showing the concentration profile of the drug in the tear fluid after rubbing application of the pharmaceutical preparation.
[FIG.7] Fig. 7 illustrates a graph showing the concentration profile of the drug in the conjunctiva after rubbing application of the pharmaceutical preparation.
[FIG. 8] Fig. 8 illustrates a graph showing the concentration profile of the drug in the plasma after rubbing application of the pharmaceutical preparation.
[FIG. 9] Fig. 9 illustrates a graph comparing the concentration profiles of the drug in the conjunctiva, between rubbing application of the solid pharmaceutical preparation and the topical instillation of eye drops.

### EXPLANATION OF SIGNS

- 1: Vertical diffusion cell
- 2: Donor cell
- 3: Donor cell compartment
- 4: Receptor cell
- 5: Receptor cell compartment
- 6: Magnetic stirrer tip
- 7: Clamp

### BEST MODE FOR CARRYING OUT THE INVENTION

In the present invention, "the surface of the skin including the surface of the eyelids" means the surface of the skin that defines the surface (front surface) of the upper and lower eyelids plus the surface of the skin adjacent to it. Though the preferred area to which the pharmaceutical preparation of the present invention is applied is the very surface of the skin defining the surface of the upper and lower eyelids, application of the preparation of the invention to the surface of the surrounding area of the skin is also allowed and preferable in order to further increase the amount of the pharmacologically active agent permeating across the skin.

In the present invention, "local tissues of the eye located on the backside of the eyelids" means the tissues which occurs in the orbit, including the cornea, bulbar conjunctiva, sclera, aqueous humor, iris, lens, ciliary body, choroid, retina, and vitreous body, as well as the palpebral conjunctiva and tear fluid.

In the present invention, when a pharmaceutical preparation is said to be "solid", it is meant that the pharmaceutical preparation lacks substantial flowability, retains its (composition's) shape, and is of such hardness that allows a user to apply it by rubbing it at its exposed portion on the skin.

According to the present invention, the components forming the base of the pharmaceutical preparation of the invention do not directly contact the anterior tissues of the eye (conjunctiva, cornea, etc.). Therefore, even such a component can be employed which cannot be included in eye drops because of irritation it would cause to the eye, insofar as it does not affect the surface of the skin. Examples of such a component include, e.g., a variety of compounds that enhance transdermal absorption of pharmacologically active ingredients. By formulating a pharmaceutical preparation employing such compounds as desired, it is possible to enhance the transdermal absorption of a pharmacologically active ingredient and thereby deliver a therapeutically needed amount of the ingredient with relative rapidity and continuously to the external tissues of the eye.

As the pharmaceutical preparation of the present invention is applied to the surface of the skin (where a number of various bacteria and fungi are commonly present), it is not necessary that the pharmaceutical preparation of the present invention be provided as a sterile preparation. Further, the preparation may be free of preservative, so long as no such composition is selected as may allow growth of bacteria or fungi.

In formulating the pharmaceutical preparation of the present invention, a variety of oily or aqueous bases may be used which form a solid containing a pharmacologically active ingredient and having such softness that allows a user to apply the preparation by rubbing it on the surface of the skin. Furthermore, there are a variety of transdermal absorption enhancers well known to those skilled in the art, from which one may choose a transdermal absorption enhancer as desired and add it to the base at a desired concentration according to the permeability of the skin to a given pharmacologically active agent to be delivered to the ocular local tissues and the concentration at which it would exhibit its pharmacological activity at the ocular local tissues.

In the present invention, examples of bases include, but are not limited to, waxes, i.e., natural waxes such as vegetable waxes (e.g., carnauba wax, Japan wax, canderilla wax), animal wax (e.g., beeswax, whale wax, wool wax), petroleum wax (e.g., paraffin wax, microcrystalline wax), mineral wax (e.g., montan wax, ozokerite), synthetic wax (e.g., carbowax), and the like, vaseline, lanoline, hydrogenated rosin/glyceryl diisostearate, hydrogenated polyisobutene, polyethylene, fatty acid cholesteryl, higher fatty acid esters (e.g., isopropyl myristate, isopropyl palmitate, oleic acid propylene glycol, hexyl laureate, decyl oleate, glyceryl monostearate), higher aliphatic alcohols (e.g., cetyl alcohol, isostearyl alcohol, lauryl alcohol, oleyl alcohol), squalene, squalane, polyethyleneglycol, higher fatty acids (e.g., isostearic acid, lauric acid, oleic acid, linoleic acid, linolenic acid), animal oils (e.g., lard), vegetable oils (e.g., castor oil), and the like, but any of the bases that can form solid pharmaceutical preparation, as well as a combination of them, may be employed. Among these bases, a particularly preferred example is a combination of an oily solid base such as waxes, vaseline or squalane with a higher fatty acid ester such as isopropyl myristate or isopropyl palmitate. Among them, higher fatty acid esters such as isopropyl myristate and isopropyl palmitate are preferable, for they not only serve as the bases to form a solid pharmaceutical preparation but also simultaneously function as an enhancer of the permeation of the skin to pharmacologically active ingredients.

Also, various other ingredients may be employed in the base in order to control the nature of the solid preparation. Examples of such ingredients include plastibase, carboxyvinylpolymer, polyacrylic acid, sodium polyacrylate, cellulose derivatives (methylcellulose, propylcellulose, hydroxyethylcellulose, hydroxypropylmethylcellulose and the like), polyvinylalcohol, polyvinylpyrrolidone, polyacrylamide, alginic acid, sodium alginate, gelatine, polysaccharide thickener (e.g., gum arabic, tragacanth gum, guar gum, xanthan gum), glycerol, sorbitol, ethylene glycol, propylene glycol and the like.

The content of oily solid bases in the pharmaceutical preparation of the present invention is preferably 15-60 wt%, more preferably 20-60 wt%, and still more preferably 35-55 wt%. For example, where a combination of waxes (animal waxes such as beeswax, and vegetable waxes such as canderilla wax) and higher fatty acid esters such as isopropyl myristate, isopropyl palmitate, and the like are employed, the content of waxes in the pharmaceutical preparation is preferably 15-60 wt%, more preferably 20-60 wt%, and still more preferably 35-55 wt%. Further, the content of higher fatty acid esters is preferably 30-60 wt%, more preferably 35-55 wt%.

The pharmaceutical preparation of the present invention may also contain a skin permeation enhancer. Examples of skin permeation enhancers include, but are not limited to, aliphatic alcohols, fatty acids and their salts, fatty acid esters, polyol alkyl ethers, glycerides, medium-chain fatty acid polyol esters, alkyl lactates, dibasic acid alkyl esters, acylated amino acids, pyrrolidones, hydroxydicarboxylic acids, or dicarboxylic acids, monoterpenes, and a variety of surfactants, and the like. Any of these skin permeation enhancers, or two or more of them, may be employed alone or in combination. Some of them are also such components that can be used in order to adjust the physical properties (e.g., hardness, viscosity, plasticity) of the base.

Examples of the above-mentioned aliphatic alcohols include ethanol, polyols such as glycerol, diethylene glycol, propylene glycol, and polyethylene glycol, and higher aliphatic alcohols. Among higher aliphatic alcohols, particularly preferred are higher aliphatic alcohols having 12-22 carbon atoms, which may be saturated or unsaturated, such as oleyl alcohol, lauryl alcohol, and the like.

Examples of the above-mentioned fatty acids and their salts include, but are not limited to, salts of capric acid, myristic acid, palmitic acid, lauric acid, stearic acid, isostearic acid, palmitoleic acid, oleic acid, vaccenic acid, linoleic acid, linolenic acid (e.g., sodium salt, potassium salt, magnesium salt, calcium salt, and aluminium salt).

Examples of the above-mentioned fatty acid esters include, but are not limited to, esters of fatty acids having 10 or more carbon atoms such as capric acid, lauric acid, palmitic acid, stearic acid, and the like with lower aliphatic alcohols having less than 12 carbon atoms such as methanol, ethanol, propanol, isopropanol, butanol, hexanol, pentanol, heptanol, and the like. Specific examples of them include isopropyl myristate, diisopropyl adipate, isopropyl palmitate, and the like.

Examples of above-mentioned polyols include, but are not limited to, ethers formed from polyols such as glycerol, ethylene glycol, propylene glycol, 1,3-butylene glycol, diglycerol, polyglycerol, diethylene glycol, polyethylene glycol, dipropylene glycol, polypropylene glycol, sorbitan, sorbitol, isosorbide, methyl glucoside, oligosaccharides, reduced oligosaccharides, and the like and alkyl alcohols (e.g., polyoxyethylene alkyl ether). The alkyl alcohol moiety of them preferably has 6-20 carbon atoms.

Preferred polyoxyethylene alkyl ethers are those consisting of an alkyl moieties having 6-20 carbon atoms and a polyoxyethylene chain having 1-9 repeating units (-O-CH₂CH₂-). Specific examples of such polyoxyethylene alkyl ethers include polyoxyethylene oleyl ether, polyoxyethylene lauryl ether, polyoxyethylene cetyl ether, polyoxyethylene stearyl ether, and the like.

With regard to glycerides, any of monoglycerides, diglycerides, and triglycerides may be employed, alone or in combination. Fatty acids as components of glycerides are preferably those having 6-18 carbon atoms. Examples of such fatty acids include octanoic acid, decanoic acid, dodecanoic acid, tetradecanoic acid, hexadecanoic acid, stearic acid, and oleic acid.

Furthermore, lactic acid, tartaric acid, 1,2,6-hexanetriol, benzyl alcohol, lanoline, potassium hydroxide, and tris (hydroxymethyl)aminomethane can also be used as skin permeation enhancers.

Examples of the above-mentioned monoterpenes include α-limonene, 1-menthol, and the like.

As the above-mentioned surfactants, anionic surfactants, cationic surfactants, nonionic surfactants and ampholytic surfactants may be employed.
Examples of anionic surfactants include salts of fatty acid, salts of alkyl sulfate, salts of polyoxyethylene alkyl sulfate, salts of alkylsulfocarbonic acid, salts of alkylethercarbonic acid, N-lauroylsarcosine and the like.
Examples of cationic surfactants include amine salts, quaternary ammonium salt, and the like.
Examples of nonionic surfactants include polyoxyethylenehydrogenated castor oil, polyoxyethylene fatty acid esters, polyoxyethylene alkyl ethers, polyoxyethylenesorbitan fatty acid esters, and the like.
Examples of ampholytic surfactants include alkylbetaine, dimethyl alkylglycine, lecithin and the like.

In addition, further examples of skin permeation enhancers include 1-dodecylazabicycloheptan-2-one, pyrrothiodecane, 2-pyrrolidone, 1-methyl-2-pyrrolidone, N,N-dimethylformamide, N,N-dimethylacetamide, dimethylsulfoxide, decylmethylsulfoxide, fumaric acid, maleic acid, myristyl lactate, cetyl lactate, lauric acid diethanolamide, and the like.

Among the above-mentioned skin permeation enhancers, particularly preferred are higher aliphatic alcohols such as lauryl alcohol and the like, fatty acids such as isostearic acid and the like, higher aliphatic esters such as isopropyl myristate, isopropyl palmitate and the like, polyoxyethylene alkyl ethers such as polyoxyethylene oleyl ether, potassium hydroxide, tris(hydroxymethyl)aminomethane and the like, and it is particularly preferred to employ a mixture of two of more of them.

Skin permeation enhancers may be included in a solid pharmaceutical preparation at any weight proportion as desired, insofar as they do not adversely affect the physical properties of the solid pharmaceutical preparation. They are included, in general, at 1-60 wt%, preferably at 10-60 wt%, more preferably at 30-60 wt% of the entire solid pharmaceutical preparation. When higher fatty acid esters such as isopropyl myristate, isopropyl palmitate and the like are employed as skin permeation enhancers, those esters are contained preferably at 30-60 wt%, and more preferably at 35-55 wt% of the entire solid pharmaceutical preparation.

For example, polyoxyethylene oleyl ethers that may be used in the present invention are those in which the average number of ethylene oxides added to the oleyl moiety is preferably 4.5-5.5, particularly preferably about 5. They may be produced by reacting, with oleyl alcohol, ethylene oxide whose hydroxy value is 4.4-5.5 as determined according to the method stipulated in the Japanese Pharmacopoeia 13th Edition, in the section of "Hydroxy Value" included in Testing Method of Fats and Fixed Oils. As such polyoxyethylene oleyl ether, Nonion E-205S produced by NOF Corporation, for example, may preferably be used.

As to pharmacologically active ingredients, drugs which have so far been topically used for ophthalmologic diseases and drugs known to be usable for the same purpose may be used also in the present invention. Such drugs include nonsteroidal antiinflammatory agents, antiallergic agents (H1 blockers), anti-glaucoma agents (α1 blockers, β-1 blockers, carbonic anhydrase inhibitors), anti-cataract agents, antimicrobial agents, antiviral agents, antifungal agents, antibiotics, sulfa agents, steroidal antiinflammatory agents, miotic agents, mydriatic agents, local astringents, vasoconstrictors, anticholinesterase agents, surface anesthetics, and vitamins (e.g., vitamin B₁₂, coenzyme-type vitamin B₂) and the like.

Specifically, they are exemplified by the following compounds: acyclovir, azulene, anthranilic acid, ascorbic acid, amlexanox, isopropyl unoprostone, idoxuridine, ibudilast, indomethacin, epinephrine, erythromycin, lysozyme hydrochloride, aprachlonidine hydrochloride, oxybuprocaine hydrochloride, carteolol hydrochloride, cyclopentrate hydrochloride, dipivephrine hydrochloride, cefmenoxime hydrochloride, dorzolamide hydrochloride, pilocarpine hydrochloride, phenylephrine hydrochloride, bunazosine hydrochloride, betaxolol hydrochloride, befunolol hydrochloride, levocabastine hydrochloride, levobunolol hydrochloride, lomefloxacin hydrochloride, ofloxacin, carbachol, dipotassium glycyrrhizinate, glutathione, sodium chromoglycate, chloramphenicol, hydrocortisone acetate, prednisolone acetate, cyanocobalamin, diclofenac sodium, distigmine bromide, homatropine hydrobromide, naphazoline nitrate, calcium diiodostearate, sulfisoxazole, sodium sulbenicillin, tazanolast, dexamethasone, tobramycin, tranilast, tropicamide, nipradilol, norfloxacin, pimaricin, pirenoxine, ketotifen fumarate, pranoprofen, flavin adenine dinucleotide, fluorometholone, prednisolone, bromfenac sodium, pemirolast potassium, helenien, timolol maleate, miopin, dexamethasone sodium metasulfobenzoate, echothiopate iodide, latanoprost, lidocaine, atropine sulfate, gentamicin sulfate, sisomicin sulfate, dibekacin sulfate, micronomicin sulfate, dexamethasone sodium phosphate, betamethasone sodium phosphate, levofloxacin, olopatadine hydrochloride, epinastine hydrochloride and the like.

Among these therapeutic agents for ophthalmologic diseases, preferred from the view point of transdermal absorption and skin permeability are those compounds having a molecular weight not more than 1000, more preferred are those having a molecular weight of not more than 800, still more preferred are those having a molecular weight of not more than 600, and particularly preferred are those having a molecular weight of not more than 500. Among those agents, preferred are antimicrobial agents, antiallergic agents, antiinflammatory agents (steroidal, non-steroidal), anti-glaucoma agents, and anti-cataract agents, with antiallergic agents and non-steroidal antiinflammatory agents being particularly preferred. Examples of them include ketotifen fumarate (antiallergic, antihistamic agent, molecular weight; 425.51), and diclofenac sodium (non-steroidal antiinflammatory agent; molecular weight 318.13).

The shape of the pharmaceutical preparation of the present invention may be as desired, e.g., short bar-like, rectangular solid-like, disk-like, and so on. It is preferably prepared as a stick-type preparation contained in a container so that the pharmaceutical preparation can be applied without directly touching it. In a particularly preferred embodiment, the pharmaceutical preparation is contained in a container which is generally cylindrical and openable at its front end, and whose bottom is formed with a movable bottom member which can be slid, by operation from outside, longitudinally in the direction toward the front end, and the solid pharmaceutical preparation is contained in the space enclosed with the cylindrical inner side surface of the container body and the movable bottom member. Such a movable bottom member may be made in any fashion as desired, and it may be of the same configuration as that of well-known conventional lipsticks or stick-type glues. For example, the movable bottom member is installed in the cylindrical container body, by being screw engaged with a through bolt extending along the central axis of the container, and engages itself with the inner shape of the side walls of the container body so as not to rotate relative to the container body. And the bolt, which is secured at its proximal end to the center of a rotary finger grip member which is attached at the proximal end of the container body and can be rotated from outside about the axis of the container body, provides, together with the rotary finger grip member, a means for translating the movable bottom member. In this case, rotation of the bolt by rotation of the rotary finger grip member causes the movable bottom member, which screw-engages with the bolt and whose rotation is blocked by the engagement with the container body, to translate longitudinally within the container body through the screw mechanism formed with the bolt.

### EXAMPLES

The present invention is described in further detail below with reference to examples. However, it is not intended that the present invention be limited to the examples.

### [Provision of a solid pharmaceutical preparation]

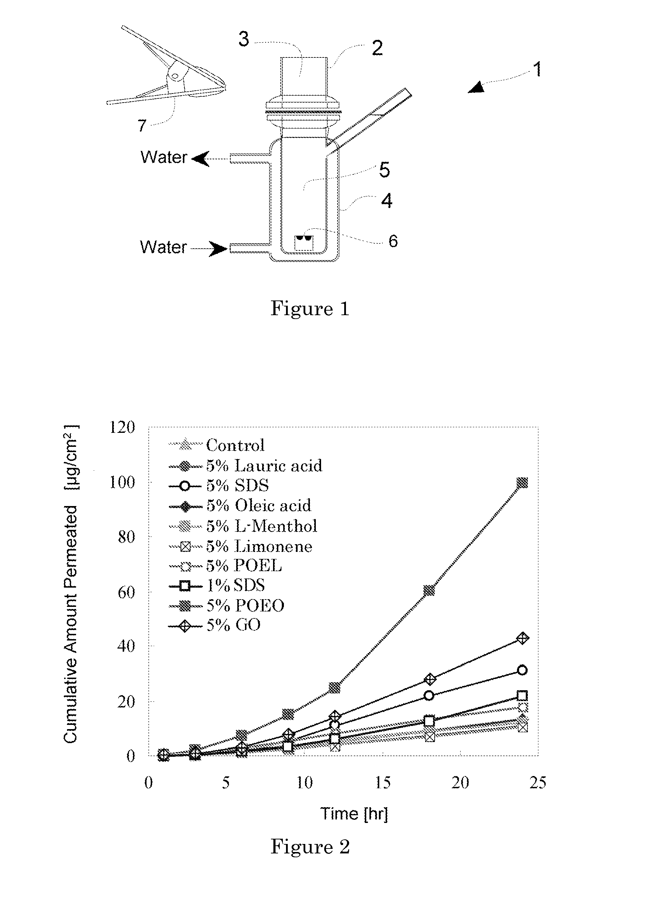

According to the formula below, ketotifen fumarate (Sigma Chemical) was chosen as an example of a pharmacologically active ingredient, and beeswax (Wako Pure Chemical Industries, Ltd.) and isopropyl myristate (Wako Pure Chemical Industries, Ltd.), which also acts as a skin permeation enhancer, as bases. As candidate skin permeation enhancers to be added to these ingredients, lauric acid, oleic acid, L-menthol, limonene, polyoxyethylene lauryl ether, sodium lauryl sulfate (all produced by Wako Pure Chemical Industries, Ltd.), polyoxyethylene oleyl ether (NOFABLE EAO-9905; Nof Corporation), and glycerol monooleate (NOF Corporation) were chosen to prepare each of the solid pharmaceutical preparations as shown in Table 1. Briefly, according to each formula, isopropyl myristate and an additional skin permeation enhancer were added to ketotifen fumarate weighed out into a beaker and stirred well. To this was added beeswax and mixed well at about 75 °C, and the mixture was quickly poured into the donor side (donor cell 2) compartment 3 of the vertical diffusion cell (Modified Franz cell) 1 illustrated in Figure 1, and let solidify in the cell, using it as a mold.

**[Table 1]**

| | Formula A1 | Formula A2 | Formula A3 | Formula A4 | Formula A5 | Formula A6 | Formula A7 | Formula A8 | Formula A9 | Formula A10 |
|---|---|---|---|---|---|---|---|---|---|---|
| Ketotifen fumarate | 4 | 4 | 4 | 4 | 4 | 4 | 4 | 4 | 4 | 4 |
| Beeswax | 51 | 46 | 46 | 46 | 46 | 46 | 46 | 50 | 46 | 46 |
| IPM | 45 | 45 | 45 | 45 | 45 | 45 | 45 | 45 | 45 | 45 |
| Lauric acid | | 5 | | | | | | | | |
| Oleic acid | | | | 5 | | | | | | |
| L-Menthol | | | | | 5 | | | | | |
| Limonene | | | | | | 5 | | | | |
| POEL | | | | | | | 5 | | | |
| SDS | | | 5 | | | | | 1 | | |
| POEO | | | | | | | | | 5 | |
| GO | | | | | | | | | | 5 |

| | | | | | | | | | | |
|---|---|---|---|---|---|---|---|---|---|---|
| Values in wt%; IPM = Isopropyl myristate, POEL = Polyoxyethylene lauryl ether, SDS = Sodium lauryl sulfate, POEO = Polyoxyethylene oleyl ether, GO = Glycerol monooleate | | | | | | | | | | |

### [In vitro skin permeation test 1]

For each of the solid pharmaceutical preparations shown in Table 1,

Referring to Figure 1, the receptor side (receptor cell 4) compartment 5 of each vertical diffusion cell 1 was filled with about 10 mL of phosphate buffer (pH 7.4) free of a drug. A piece of intact abdominal skin of a hairless mouse was cut out and, and fixed to cover the opening of each receptor cell 4, with its outer surface facing upward (effective diffusion area: 1.72 cm²). At this position, the lower surface of the skin was allowed constantly to contact the phosphate buffer in the receptor cell 4. The donor cell 2 was mounted on the skin fixed over the opening of the receptor cell 4, with one of the solid pharmaceutical preparations of the above-mentioned formulas A1 to A10 (of these, A1 is the control), which had been formed using the donor side compartment 3 as a mold, protruding downwardly by about 1 mm from the opening of the donor cell 2, and was secured there with a clamp 7. Then, skin permeability experiments were started. Constantly stirring the buffer with a magnetic stirrer tip 6, 200-µl sampling was performed at predetermined intervals from the receptor cell 4, while immediately supplementing the same amount of the drug-free phosphate buffer to keep the volume of the receptor solution constant. During the experiment, the temperature was maintained at 37 °C. Determination of ketotifen fumarate in the samples was performed by HPLC under the following conditions. The results are shown in Figure 2 and Table 2.

### <HPLC conditions>

Detector: UV spectrophotometer (detection wavelength 300 nm)
Column: Capcell pak C18 MG S5 µm, 4.5 × 250 mm (manufactured by Shiseido)
Guard column: TSK-GEL ODS-80Ts (manufactured by Tosoh Corporation)
Column temp.: constant temperature at about 40 °C
Mobile phase: 0.1 M Tris(hydroxymethyl)aminomethane) (manufactured by Wako Pure Chemical Industries) buffer (pH 9)/acetonitrile = 30:70
Flow rate: 1.0 mL/min
Injection volume: 30 µL

**[Table 2]**

| | Formula A1 | Formula A2 | Formula A3 | Formula A4 | Formula A5 | Formula A6 | Formula A7 | Formula A8 | Formula A9 | Formula A10 |
|---|---|---|---|---|---|---|---|---|---|---|
| dQ/dt | 0.63 | 0.52 | 1.72 | 0.67 | 0.62 | 0.53 | 0.89 | 1.25 | 6.23 | 2.34 |
| td | 3.17 | 3.63 | 5.72 | 4.42 | 4.04 | 4.7 | 2.94 | 7.04 | 8.14 | 5.91 |

| | | | | | | | | | | |
|---|---|---|---|---|---|---|---|---|---|---|
| dQ/dt [µg/cm²/hr] = rate of skin permeation of a drug per unit time, td [hr] = time delay | | | | | | | | | | |

In Figure 2 (Q-t time curve), the axis of ordinate represents cumulative amount permeated per unit area (Q), and the axis of abscissas represents time (t). In Table 2, time delay (td [hr]) is the time intercept with the extension of the linear portion of the Q-t time curve in Figure 2. dQ/dt represent the amount of permeated pharmacologically active ingredient (µg) per unit area (1 cm²) per unit time (1 hr). As evident from Table 2 and Figure 2, while continuous permeation of ketotifen fumarate to the receptor side cell was observed with any of the solid pharmaceutical preparation, remarkable increase in skin permeation was noted with solid preparation containing sodium lauryl sulfate, glycerol monooleate or polyoxyethylene oleyl ether in addition to isopropyl myristate, compared with the solid preparation of formula A1 (control), which contained only one skin permeation enhancer, isopropyl myristate, which also acted as a base. Among others, polyoxyethylene oleyl ether was found to be very effective.

### [In vitro skin permeation test 2]

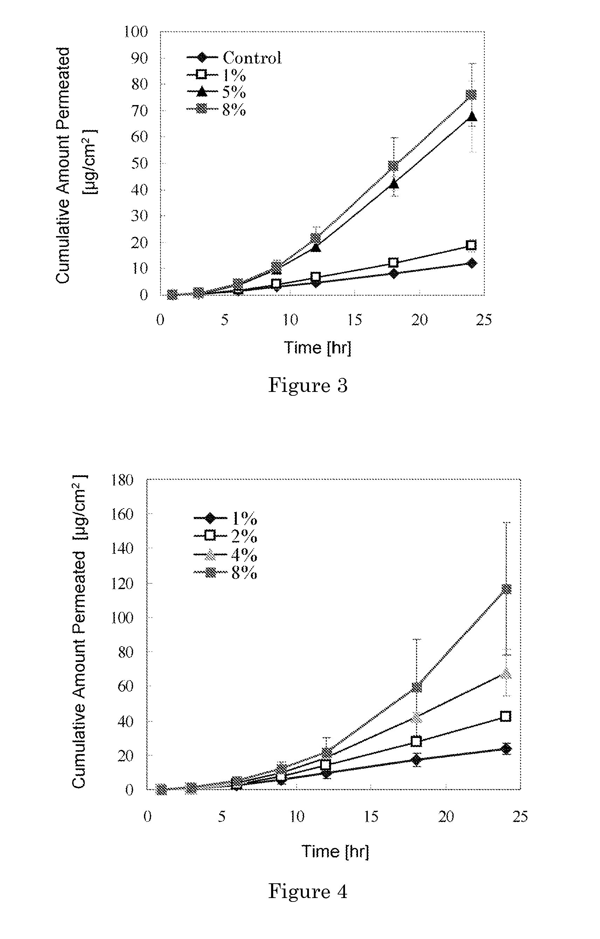

In order to closely examine the effect of polyethylene oleyl ether at different concentrations on the skin permeation of pharmacologically active ingredients, solid pharmaceutical preparations containing polyoxyethylene oleyl ether at different concentrations (0, 1, 5 and 8 wt%) were prepared in accordance with the formulas set forth in Table 3, and skin permeation rates were measured as in the above test. The results are shown in Figure 3 and Table 4. Formula B1 is the control in this test. In the following test examples, Nonion E-205S (Yuka Sangyo Co. Ltd.) was used as polyoxyethylene oleyl ether (POEO).

**[Table 3]**

| | Formula B1 | Formula B2 | Formula B3 | Formula B4 |
|---|---|---|---|---|
| Ketotifen fumarate | 4 | 4 | 4 | 4 |
| Beeswax | 51 | 50 | 46 | 43 |
| IPM | 45 | 45 | 45 | 45 |
| POEO | 0 | 1 | 5 | 8 |

**[Table 4]**

| | Formula B1 | Formula B2 | Formula B3 | Formula B4 |
|---|---|---|---|---|
| dQ/dt | 0.59 ± 0.07 | 1.02 ± 0.15 | 4.12 ± 1.19 | 4.46 ±0.72 |
| td | 3.90 ± 1.39 | 5.51 ± 1.07 | 7.26 ± 1.75 | 7.06 ± 0.29 |

| | | | | |
|---|---|---|---|---|
| n=3, Values representing mean ± standard deviation | | | | |

As evident from Figures 3 and 4, the polyoxyethylene oleyl ether-induced increase in the skin permeation rate of the pharmacologically active ingredient was dose-dependent, with remarkable acceleration of the skin permeation rate of the pharmacologically active ingredient using polyoxyethylene oleyl ether at a concentration between 1 wt% and 5 wt%.

### [In vitro skin permeation test 3]

In order to examine the influence of the concentration of a pharmacologically active ingredient on the skin permeation of the very pharmacologically active ingredient, solid pharmaceutical preparations of the formulas shown in Table 5 were prepared with varying concentrations of ketotifen fumarate, and their skin permeation was tested in vitro in the same manner as in the above tests. The results are shown in Figure 4 and Table 6.

**[Table 5]**

| | Formula C1 | Formula C2 | Formula C3 | Formula C4 |
|---|---|---|---|---|
| Ketotifen fumarate | 1 | 2 | 4 | 8 |
| Beeswax | 49 | 48 | 46 | 42 |
| IPM | 45 | 45 | 45 | 45 |
| POEO | 5 | 5 | 5 | 5 |

**[Table 6]**

| | Formula C1 | Formula C2 | Formula C3 | Formula C4 |
|---|---|---|---|---|
| dQ/dt | 1.27 ± 0.21 | 2,24 ± 0.16 | 4.12 ± 1.19 | 7.90 ± 2.51 |
| td | 4.29 ± 1.85 | 5.73 ± 0.36 | 7.26 ± 1.75 | 9.75 ±0.53 |

| | | | | |
|---|---|---|---|---|
| n=3, values representing mean ± standard deviation | | | | |

As seen in Figure 4 and Table 6, as the concentration of ketotifen fumarate in the solid pharmaceutical preparation was increased, from 1 wt% to 2, 4 and 8 wt%, its skin permeation rate increased roughly parallel to the concentration of ketotifen fumarate, i.e., 1.76 folds at 2 wt%, 3.24 folds at 4 wt%, and 6.22 folds at 8 wt%, as compared with the 1 wt% formula.

### [In vitro skin permeation test 4]

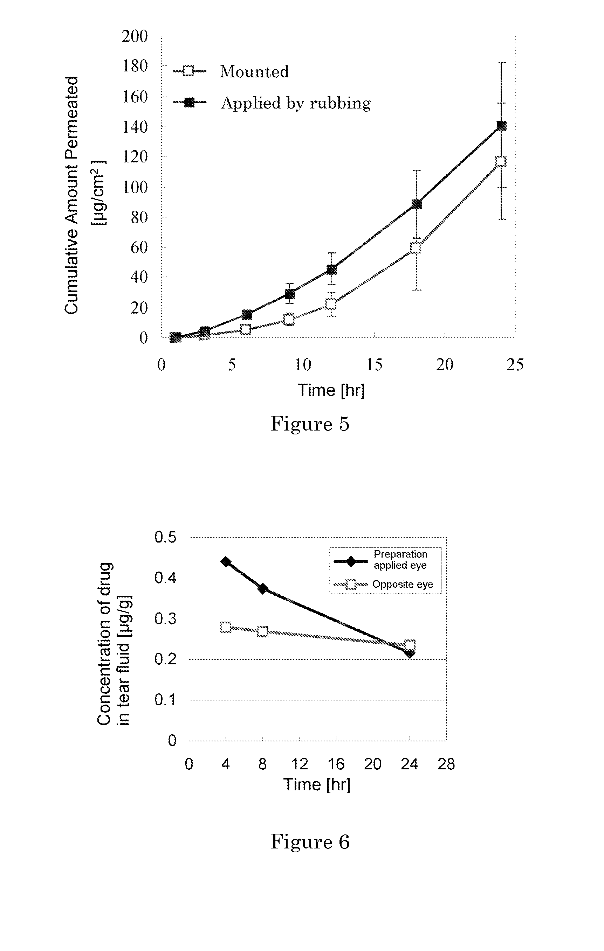

Skin permeation of a pharmacologically active ingredient of a solid pharmaceutical preparation containing the same, when applied by rubbing the preparation on the skin, was compared with the skin permeation achieved when the solid pharmaceutical preparation was mounted on the skin. According to Formula C4 in Table 5, isopropyl myristate and polyoxyethylene oleyl ether were added to ketotifen fumarate weighed out in a beaker, and stirred well. To this was added beeswax, and mixed well at about 75 °C. The mixture was then poured portionwise into a lip tube (volume: 5 mL, H68.0 mm × (ϕ16.0 mm) which had been adjusted to have a void of about 15 mm in depth from the top of the container (about 2 mL in volume) by rotating its screw, and was allowed to solidify at room temperature to afford a cylindrical ophthalmic solid pharmaceutical preparation for external use. Skin permeation of ketotifen fumarate was examined under two conditions, in one of which the solid pharmaceutical preparation was applied in a sufficient amount from the lip tube by rubbing it ten times on an excised skin of a mouse (applied to the area of about 4 cm²), and the skin was set on the receptor cell, and in the other of which the solid pharmaceutical preparation was placed on the skin in the same manner as in the above-mentioned tests. The results are shown in Figure 5.

Figure 5 shows that the skin permeation rate of ketotifen fumarate where the solid pharmaceutical preparation was rubbed on the skin surface was not less than that where the solid pharmaceutical preparation was placed on the skin surface.

### [In vitro skin permeation test 5]

Skin permeation rates were compared between two solid pharmaceutical preparations whose bases differed from each other. Briefly, according to the formulas in Table 7, solid pharmaceutical preparations were prepared in lip tubes (volume: 5 mL, H68.0 mm × ϕ16.0 mm) and rubbed on sheets of excised mouse skin, which then were fixed on receptor cells to measure the skin permeation rates of ketotifen fumarate. The results are shown in Table 8.

**[Table 7]**

| | Formula C3 | Formula D1 |
|---|---|---|
| Ketotifen fumarate | 4 | 4 |
| Beeswax | 46 | 15 |
| Canderilla wax | - | 5 |
| Squalane | - | 26 |
| IPM | 45 | 45 |
| POEO | 5 | 5 |

**[Table 8]**

| | Formula C3 | Formula D1 |
|---|---|---|
| dQ/dt | 4.25 ± 0.29 | 4.74 ± 1.04 |
| td | 4.27 ± 2.10 | 4.13 ± 0.73 |

As seen in Table 8, the both solid pharmaceutical preparations exhibited continuous high skin permeation. Further, there were found no difference between the two formulas, indicating that various oily bases may be employed which is capable of forming solid pharmaceutical preparations having proper hardness for easy application by rubbing.

### [In vivo drug transfer test]

A test was performed as follows to examine whether a pharmacologically active ingredient could be transferred to ocular tissues after application by rubbing of a solid pharmaceutical preparation containing a pharmacologically active ingredient on the surface of the skin including the front surface of the eyelids of rabbits.

### <Provision of a solid pharmaceutical preparation>

Using ketotifen fumarate as the pharmacologically active ingredient, a cylindrical stick-type solid pharmaceutical preparation was prepared in a lip tube according to the following formula.

**(Formula C4)**

| | |
|---|---|
| Ketotifen fumarate | 8 g |
| Beeswax | 42 g |
| Isopropyl myristate | 45 g |
| Polyoxyethylene oleyl ether | 5 g |
| Total amount | 100 g |

To ketotifen fumarate weigh out into a beaker were added isopropyl myristate and polyoxyethylene oleyl ether and mixed well. To this was added beeswax and mixed well at about 75 °C. Then it was poured portionwise into a lip tube (volume: 5 mL, H68.0 mm × ϕ16.0 mm) and allowed to solidify at room temperature to afford a stick-type solid pharmaceutical preparation.

### <Administration of the solid pharmaceutical preparation>

On the day before the test, rabbits were subjected to removal of hair around their upper and lower eyelids on both eyes. The rabbits held in holders and their skin area of 3.5-4 cm² was marked below one of the eye including the lower eyelid, and the above solid pharmaceutical preparation was then applied by rubbed it ten times on that area. The net amount of ketotifen fumarate applied was about 14.4 mg as calculated based on the reduced amount of the solid pharmaceutical preparation and the proportion of each component of the preparation.

### <Sampling of tissues>

At 4, 8 and 24 hours after the application of the solid pharmaceutical preparation, the pharmaceutical preparation remaining on the surface of the skin was softly wiped out. In order to avoid contamination with the pharmaceutical preparation still remaining on the skin, the area to which the application had been made was covered with a tape, and the tear fluid then was collected using a capillary. After sampling of the blood, the rabbits were euthanized with an excess amount of a pentobarbital sodium solution. The collected blood was centrifuged for about 5 minutes, and the plasma was transferred to Eppendorf tubes. The anterior segments of the eye were washed with saline, and the pharmaceutical preparation was wiped out from the skin of the lower eyelid, and the eyeball was taken out with attached conjunctiva. The conjunctiva was taken from the excised eye on a sheet of filter paper. The conjunctiva thus taken was frozen stored at -80 °C.

### <Measurement of ketotifen fumarate in the tissues>

(1) Measurement of concentration in the tear fluid: The wet weight of the tear fluid collected in an Eppendorf tubes was measured. With addition of the mobile phase to them, samples in three tubes were combined into one and the total amount of about 160 µL was ultracentrifuged at 14000 rpm for five minutes. One hundred and fifty µL of the supernatant was transferred into a vial, and 50 µL of it was injected into a HPLC for quantitative measurement.
(2) Measurement of concentration in the conjunctiva: The wet weight of the conjunctiva taken in a spitz tubes was measured. To this was added 1 mL of 10 mM sodium dihydrogen phosphate buffer (pH 7.0) and the conjunctiva was minced. Further, 4 mL of acetonitrile was added, and the mixture was shaken at 300 rpm for 10 minutes. Following centrifugation at 3000 rpm for 10 minutes, 4 mL each of the supernatant was taken in separate test tubes for concentration and let dry under reduced pressure for about 18 hours in a centrifuge evaporator (the samples in 3 tubes were finally combined into one during concentration). Then, this was dissolved again by addition of 300 µL of the mobile phase, and the total volume was transferred into an Eppendorf tube. After ultracentrifugation for 5 minutes at 14000 rpm, the supernatant was filtered through a membrane filter (0.22 µm). The filtrate was transferred into a vial, and 50 µL of it was injected into a HPLC for quantitative measurement.
(3) Measurement of concentration in the plasma: Of the plasma taken into the Eppendorf tube, 1 mL was transferred into a test tube, and 1 mL of 10 mM sodium dihydrogen phosphate buffer (pH 7.0) was added, and the mixture stirred. Further, 4 mL of acetonitrile was added, and the mixture was shaken at 300 rpm for 10 minutes. Following centrifugation at 3000 rpm for 10 minutes, 4 mL each of the supernatant was taken into separate test tubes for concentration and let dry under reduced pressure for about 18 hours in a centrifuge evaporator (the samples in 3 tubes were finally combined into one during concentration). Then, this was dissolved again by addition of 500 µL of the mobile phase, and the total volume was transferred into an Eppendorf tube. After ultracentrifugation for 5 minutes at 14000 rpm, the supernatant was filtered through a membrane filter (0.22 µm). The filtrate was transferred to a vial, and 50 µL of it was injected into a HPLC for quantitative measurement.

The concentration of ketotifen fumarate in the tear fluid, conjunctiva and the plasma 4, 8 and 24 hours after rubbing application of the solid pharmaceutical preparation is shown in Figures, 6, 7, and 8 and Tables 9, 10 and 11, respectively.

**[Table 9]**

| Time after application (hr) | Concentration in tear fluid [µg/g] | |
|---|---|---|
| | Preparation administered eye | Opposite eye Opposite eye |
| 4 | 0.440 | 0.279 |
| 8 | 0.374 | 0.269 |
| 24 | 0.216 | 0.235 |

**[Table 10]**

| Time after application (hr) | Concentration in conjunctiva [µg/g] | |
|---|---|---|
| | Preparation administered eye | Opposite eye |
| 4 | 0.353 | 0.173 |
| 8 | 0.598 | 0.123 |
| 24 | 0.447 | 0.151 |

**[Table 11]**

| Time after application (hr) | Concentration in plasma [µg/g] Concentration in |
|---|---|
| 4 | 0.046 |
| 8 | 0.030 |
| 24 | 0.017 |

As seen in Figures 6-8 and Tables 9-11, after rubbing application of the solid pharmaceutical preparation, the concentration of the drug in the tear fluid on the preparation-applied side reached 0.440 µg/g in 4 hours, and it, though reduced thereafter, still remained at a value of 0.216 µg/g even 24 hours after the application. On the opposite side, to which no solid pharmaceutical preparation was applied, the concentration of the drug in the tear fluid reached about 60 % of the preparation-applied side at 4 hours after the application, and it gradually reduced thereafter and became, at 24 hours after application, comparable to that on the preparation-applied side.
The concentration of the drug in the conjunctiva (lower palpebral conjunctiva) was 0.353 µg/g at 4 hours after rubbing application in the solid preparation-applied eye, which was somewhat lower than the concentration of the drug in the tear fluid, reached a high value of 0.598 µg/g at 8 hours after the application, and still remained at a value of 0.447 µg/g even at 24 hours after the application. In contrast, the concentration of the drug in the conjunctiva of the opposite eye was constantly on the order of 0.1 µg/g, without showing any significant fluctuation.
The concentration of the drug in the plasma was 0.046 µg/mL at 4 hours after the application, and reduced to 0.030 µg/mL at 8 hours and then to 0.017 µg/ml at 24 hours after the application.

The above data were fed into SKIN-CAD^{®} (Biocom Systems, Inc., Fukuoka, Japan), a software for analysis of skin permeation of drugs, together with the parameters for hairless mouse skin permeation and parameters for rabbit tear fluid kinetics, to simulate a ketotifen fumarate concentration profile in rabbit's conjunctiva. The parameters employed are shown in Table 12.

**[Table 12]**

| - Parameters - | Values |
|---|---|
| Duration of Medication [hr] | 24, 1 Dose |
| Duration of Transdermal Treatment System Application | 24, 1 Dose |
| Transdermal Treatment System Size (Surface Area) [cm²] | 4 |
| - Device Design - | |
| Thickness of Device [cm] | 0.05 |
| Diffusion Coefficient in Device [cm²/sec] | 1.97 × 10⁻⁹ |
| Initial Drug Concentration in Device [µg/mL] | 1.44 × 10⁴ |
| Device/Skin Partition Coefficient [-] | 5.80 × 10⁻² |
| - Skin Structure - | |
| Thickness of Stratum Corneum [cm] | 0.00075 |
| Thickness of Whole Skin [cm] | 0.125 |
| Distance to Blood Vessel [cm] | 0.0075 |
| Absorption Rate Constant into Blood [1/sec] | 4.00 × 10⁻² |
| - Skin Permeation - | |
| Diffusion Coefficient in Stratum Corneum [cm^{2/}sec] | 1.12 × 10⁻¹¹ |
| Diffusion Coefficient in Viable Skin [cm²/sec] | 3.75 × 10⁻⁷ |
| Stratum Corneum/Viable Skin Partition Coefficient [-] | 2.47 × 10² |
| - Tear Fluid Pharmacokinetics - | |
| Volume of Distribution, V1 [mL] | 8.00 × 10⁻³ |
| Elimination Rate Constant, K10 [1/sec] | 3.85 × 10⁻³ |

As a result of the simulation, it was found that expected values, when assuming that 3 % of ketotifen fumarate administered was transferred to the tear fluid, well agree with the values obtained in the experiment. According to references, the rate of uptake of absorbed drugs by the subvascular tissues in normal capillary vessels is around 3-5 % (K. Tojo, Mathematical models of transdermal and topical drug delivery, 2nd edition, Biocom Systems, Fkuoka, Japan, 2005), and the above transfer rate of 3 % (i.e., the rate of uptake into the tear fluid) almost agrees with this. Based on this rate of uptake (3 %), the profile of drug concentration in the conjunctiva was simulated again, this time on the assumption that the solid pharmaceutical preparation was rubbed on the skin only once, not ten times, by replacing the parameter for drug thickness in SKIN-CAD^{®} with 1/10 of the value in the above test, i.e., with 0.005 cm.

Besides, Table 13 sets forth the data showing the ketotifen fumarate concentration in the conjunctiva after topical instillation of ketotifen fumarate (0.967 W/V%) eye drops to rabbit eyes (source: Distribution of 14C-ketotifen fumarate in Rabbit Eye Tissue after Ophthalmic Administration, Shinichi Ota, Journal of Clinical Therapeutics and Medicines, 4(11), 1988).

**[Table 13]**

| Tim after topical instillation (hr) | Concentration in conjunctiva [µg/g] | |
|---|---|---|
| | Mean | Standard deviation. |
| 0.25 | 2.683 | 1.098 |
| 0.5 | 0.558 | 0.065 |
| 0.75 | 0.424 | 0.229 |
| 1 | 0.246 | 0.075 |
| 2 | 0.036 | 0.009 |
| 3 | 0.032 | 0.015 |
| 4 | 0.045 | 0.014 |
| 6 | 0.028 | 0.003 |
| 8 | 0.022 | 0.001 |
| 24 | 0.028 | 0.013 |

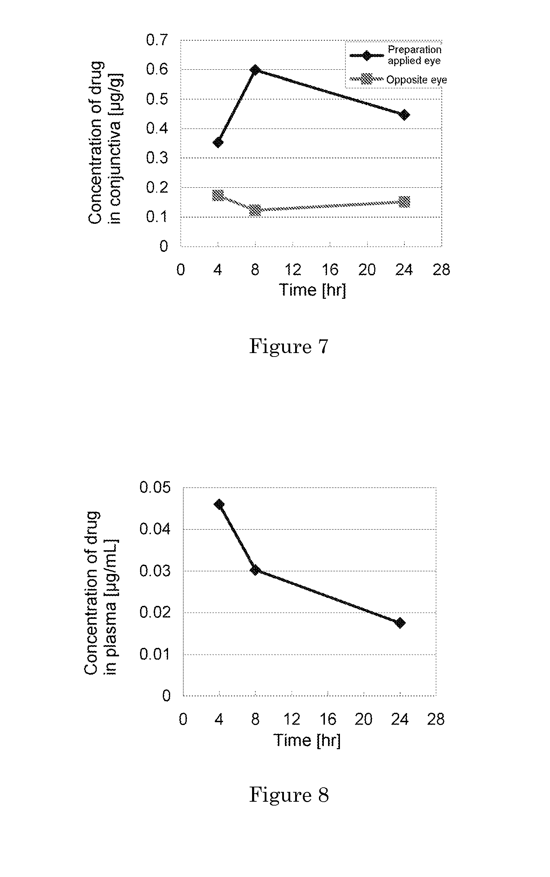

As evident from Tables 10 and 13, the concentration of the drug in the conjunctiva is high at 0.25 hour after the topical instillation, but sharply reduces thereafter, i.e., to about 1/5 at 0.5 hour after the topical instillation, and 1/10 at one hour after the topical instillation. In contrast, the conjunctival concentration of the drug after the rubbing application of the solid pharmaceutical preparation, as shown in Table 10 and Figure 7, reached its high value at 8 hours after the application and remained high for a remarkably extended length of time, instead of lacking a sharp increase immediately after the application.

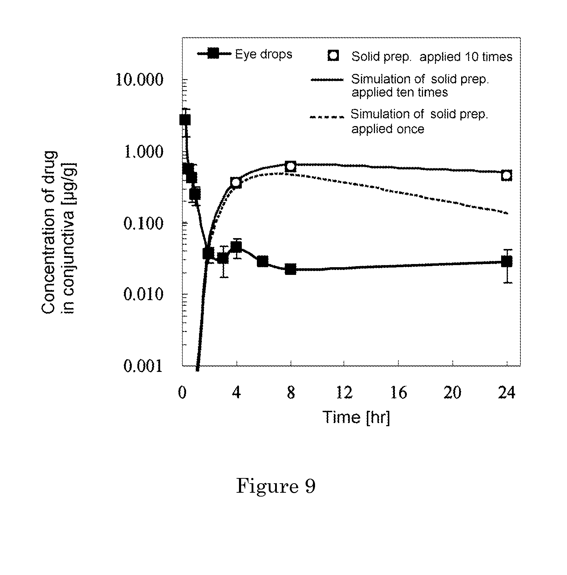

As to the concentration of the drug in the conjunctiva, the results of the above test where the solid pharmaceutical preparation was applied (Table 10, 10 times application) and a graph produced by SKIN-CAD^{®} in agreement with the results, the data after the above topical instillation (Table 13), and the result of the simulation by SKIN-CAD^{®} where the solid pharmaceutical preparation was rubbed only once (1 time application) are shown together in Figure 9.

Figure 9 shows that, in contrast to topical instillation, after the solid pharmaceutical preparation applied (10 times), the concentration of the drug in the conjunctiva is maintained for a very long time that exceeds 24 hours. In Figure 9, the simulation graph of one time application of the solid pharmaceutical preparation indicates that even only one-time application of the solid pharmaceutical preparation can achieve a remarkably long-lasting and sustained conjunctival drug concentration compared with topical instillation. Taken together, these results show that rubbing application of a solid pharmaceutical preparation enables much longer maintenance of drug concentrations in ocular local tissues, compared with topical instillation.

### [Preparation Example 1]

| | |
|---|---|
| Ketotifen fumarate | 8 g |
| Beeswax | 42 g |
| Isopropyl myristate | 45 g |
| Polyoxyethylene oleyl ether | 5 g |
| Total amount | 100 g |

To ketotifen fumarate weight out into a beaker are added isopropyl myristate and polyoxyethylene oleyl ether, and stirred well. Beeswax is added to this and mixed well at about 75 °C. The mixture was then poured portionwise into a predetermined mold and allowed to solidify to afford a solid pharmaceutical preparation.

### [Preparation Example 2]

| | |
|---|---|
| Ketotifen fumarate | 8 g |
| Beeswax | 14 g |
| Canderilla wax | 5 g |
| Squalane | 23 g |
| Isopropyl myristate | 45 g |
| Polyoxyethylene oleyl ether | 5 g |
| Total amount | 100 g |

To ketotifen fumarate weight out into a beaker are added squalane, isopropyl myristate and polyoxyethylene oleyl ether and stirred well. Beeswax and canderilla wax are added to this and mixed well at about 75 °C. Then the mixture is poured portionwise into a predetermined mold and allowed to solidify to afford a solid pharmaceutical preparation.

### [Preparation Example 3]

| | |
|---|---|
| Ketotifen fumarate | 10 g |
| Vaseline | 40 g |
| Lauryl alcohol | 5 g |
| Isopropyl palmitate | 45 g |
| Polyoxyethylene oleyl ether | 5 g |
| Total amount | 100 g |

To ketotifen fumarate weighed out into a beaker are added isopropyl myristate and polyoxyethylene oleyl ether and mixed well. Lauryl alcohol is further added and mixed well. To this is added vaseline at about 60 °C and mixed well. Then the mixture is poured portionwise into a predetermined mold to solidify to afford a solid pharmaceutical preparation.

### [Preparation Example 4]

| | |
|---|---|
| Diclofenac sodium | 10 g |
| Vaseline | 40 g |
| Isopropyl palmitate | 45 g |
| Sodium lauryl sulfate | 5 g |
| Total amount | 100 g |

To diclofenac sodium weighed out into a beaker are added isopropyl palmitate and sodium lauryl sulfate and mixed well. To this is added vaseline and mixed well at about 60 °C. Then, the mixture is poured portionwise into a predetermined mold to afford a solid pharmaceutical preparation.

### [Preparation Example 5]

| | |
|---|---|
| Pilocarpine hydrochloride | 5 g |
| Canderilla wax | 35 g |
| Isopropyl myristate | 55 g |
| Glycerol monooleate | 5 g |
| Total amount | 100 g |

### [Preparation Example 5]

To pilocarpine hydrochloride weighed out into a beaker are added isopropyl myristate and glycerol monooleate and mixed well. To this is added canderilla wax and mixed well at about 75 °C. Then, the mixture is poured portionwise into a predetermined mold and allowed to solidify to afford a solid pharmaceutical preparation.

### [Preparation Example 6]

| | |
|---|---|
| Olopatadine hydrochloride | 5 g |
| Beeswax | 55 g |
| Isopropyl myristate | 35 g |
| Polyoxyethylene oleyl ether | 5 g |
| Total amount | 100 g |

To olopatadine hydrochloride weight out into a beaker are added isopropyl myristate and polyoxyethylene oleyl ether and mixed well. To this is added beeswax and mixed well at about 75 °C. Then, the mixture is poured portionwise into a predetermined mold and allowed to solidify to afford a solid pharmaceutical preparation.

### [Preparation Example 7]

| | |
|---|---|
| Epinastine hydrochloride | 10 g |
| Beeswax | 40 g |
| Isopropyl myristate | 45 g |
| Polyoxyethylene oleyl ether | 5 g |
| Total amount | 100 g |

To epinastine hydrochloride weight out into a beaker are added isopropyl myristate and polyoxyethylene oleyl ether and mixed well. To this is added beeswax and mixed well at about 75 °C. Then, the mixture is poured portionwise into a predetermined mold and allowed to solidify to afford a solid pharmaceutical preparation.

### INDUSTRIAL APPLICABILITY

The present invention can be utilized to provide a novel form of ophthalmic pharmaceutical preparation for external use having such an excellent property that it enables continuous supply of a pharmacologically active agent to ocular tissues for an extended length of time.

## Claims

1. An ophthalmic solid pharmaceutical preparation for external use comprising a base containing a pharmacologically active ingredient, wherein the solid pharmaceutical preparation is designed to be applied by rubbing on the surface of the skin including the surface of either of the eyelids to deliver, through the skin of the eyelids, the pharmacologically active ingredient to ocular local tissues located on the backside of the eyelids.

2. The ophthalmic solid pharmaceutical preparation for external use according to claim 1, wherein the base comprises an oily solid base.

3. The ophthalmic solid pharmaceutical preparation for external use according to claim 2, wherein the oily solid base is wax or vaseline.

4. The ophthalmic solid pharmaceutical preparation for external use according to claim 3, wherein the wax is animal wax or vegetable wax.

5. The ophthalmic solid pharmaceutical preparation for external use according to one of claims 1 to 4, wherein the preparation contains a skin permeation enhancer.

6. The ophthalmic solid pharmaceutical preparation for external use according to claim 5, wherein the skin permeation enhancer is selected from the group consisting of an ester made from a fatty acid and a lower aliphatic alcohol, and a surfactant.

7. The ophthalmic solid pharmaceutical preparation for external use according to claim 6, wherein the content of the ester is 30-60 wt%.

8. The ophthalmic solid pharmaceutical preparation for external use according to claim 6 or 7, wherein the ester is isopropyl myristate or isopropyl palmitate, and wherein the surfactant is a nonionic surfactant.

9. The ophthalmic solid pharmaceutical preparation for external use according to one of claims 1 to 8, wherein the base comprises an oily solid base, wherein the content of the oily solid base in the pharmaceutical preparation is 15-60 wt%.

10. The ophthalmic solid pharmaceutical preparation for external use according to one of claims 1 to 9, wherein the pharmacologically active ingredient is selected from the group consisting of non-steroidal antiinflammatory agents, antiallergic agents, anti-glaucoma agents, anti-cataract agents, antimicrobial agents, antiviral agents, antifungal agents, antibiotics, sulfa agents, steroidal antiinflammatory agents, miotic agents, mydriatic agents, local astringents, vasoconstrictors, anticholinesterases, surface anesthetics, and vitamins.

11. The ophthalmic solid pharmaceutical preparation for external use according to one of claims 1 to 10, wherein the shape of the pharmaceutical preparation is of a short bar.

12. A stick type ophthalmic preparation comprising a generally cylindrical container body defining one openable end, a movable bottom member which is fit in the interior of the container body and can be translated in the direction toward the openable end, and a drive means to cause the movable bottom member to translate in the direction toward the open end, wherein the ophthalmic solid pharmaceutical preparation for external use according to one of (1) to (11) above is contained, in the container body, between the openable end and the movable bottom member, wherein the ophthalmic solid pharmaceutical preparation for external use can be projected from the container body as a result of the forward translation of the movable bottom member driven by the drive means.

13. A method for delivering a pharmacologically active ingredient to the ocular local tissues located on the backside of the eyelids, the method comprising rubbing an ophthalmic solid pharmaceutical preparation for external use comprising a base containing a pharmacologically active ingredient, on the surface of the skin including the surface of either of the eyelids to apply the preparation.

14. The method according to claim 13, wherein the base comprises an oily solid base.

15. The method according to claim 14, wherein the oily solid base is wax or vaseline.

16. The method according to one of claims 13 to 15, wherein the ophthalmic solid pharmaceutical preparation for external use contains a skin permeation enhancer.

17. Use of an oily solid base for the manufacture of an ophthalmic solid pharmaceutical preparation for external use containing a pharmacologically active ingredient for delivering the pharmacologically active ingredient to the tissues located on the backside of the eyelids.

18. Use of an oily solid base and a skin permeation enhancer for the manufacture of an ophthalmic solid pharmaceutical preparation for external use containing a pharmacologically active ingredient for delivering the pharmacologically active ingredient to the tissues located on the backside of the eyelids.

19. The use according to claim 17 or 18, wherein the oily solid base is wax or vaseline.
